# FASCICULE DE BREVET EUROPEEN

(11) **EP 2 825 097 B1**
(45) Date de publication et mention de la délivrance du brevet: **29.08.2018**
(21) Numéro de dépôt: 13710494.9
(22) Date de dépôt: 21.02.2013
(51) Int. Cl.: A61B 5/22

(54) **DISPOSITIF D'EVALUATION DE LA MOTRICITE DISTALE DES MEMBRES SUPERIEURS D'UN INDIVIDU.**
VORRICHTUNG ZUR BEWERTUNG DER DISTALEN MOTORISCHEN FERTIGKEITEN DER OBEREN EXTREMITÄTEN EINER PERSON
DEVICE FOR EVALUATING THE DISTAL MOTOR SKILLS OF THE UPPER LIMBS OF A PERSON

(30) Priorité: 21.02.2012 FR 1251540
(43) Date de publication de la demande: 21.01.2015
(73) Titulaire: Association Institut de Myologie, 75013 Paris (FR)
(72) Inventeur: HOGREL, Jean-Yves, 92120 Montrouge (FR); SERVAIS, Laurent, 75012 Paris (FR); CANAL, Aurélie, 75013 Paris (FR); VOIT, Thomas, 91470 Boullay-les-Troux (FR)
(74) Mandataire: Gauchet, Fabien Roland
(86) Numéro de dépôt international: PCT/FR2013/050353
(87) Numéro de publication internationale: WO 2013/124586

(56) Documents cités:
- WO-A2-01/72223
- FR-A1- 2 961 086
- US-A1- 2004 097 838
- PATAKY ET AL: "A Device for Testing the Intrinsic Muscles of the Hand", JOURNAL OF HAND THERAPY, HANLEY AND BELFUS, US, vol. 20, no. 4, 22 octobre 2007 (2007-10-22), pages 345-350, XP022308181, ISSN: 0894-1130 cité dans la demande

## Description

### DOMAINE TECHNIQUE DE L'INVENTION

L'invention se rapporte au domaine de l'évaluation de la capacité motrice des membres supérieurs d'un individu.

Plus particulièrement il s'agit de détecter, de mesurer, de mémoriser et de traiter des mesures relatives à la force et à la fonction musculaire d'un individu notamment à la capacité motrice des fonctions de flexion/extension du poignet et des doigts d'un individu.

L'évaluation de la capacité musculaire est particulièrement intéressante dans le cas du diagnostic de pathologies touchant les muscles telles que les myopathies, les pathologies neurologiques, rhumatismales, traumatiques, la sarcopénie liée à l'âge. Cette évaluation permet avantageusement d'évaluer les effets thérapeutiques d'un traitement ou d'une intervention ; les mesures des capacités motrices indiquées ci-dessus sont le premier et principal maillon de telles évaluations.

### ETAT DE LA TECHNIQUE ANTERIEURE

Dans le document FR 2 961 086 on expose un dispositif d'évaluation de la capacité motrice des membres supérieurs d'un individu, qui comprend une tablette munie d'une rainure, un module de mesure mobile en translation le long de la rainure, deux boutons poussoirs (touches) ayant chacun une face d'appui de hauteur différente et variable par rapport à la surface de la tablette et étant chacun couplé à un capteur de force apte à générer un signal corrélé à la force de l'individu qui appuie sur le bouton ; un système de contrôle est par ailleurs couplé au module de mesure et il comprend un compteur d'actions réalisées par l'individu sur chacune des touches ainsi qu'un chronomètre intégré et programmable permettant un décompte temporel.

Ce dispositif remplit la fonction de base qui lui est dévolue à savoir détecter et compter les appuis marqués d'un ou de plusieurs doigt d'un individu sur chacune des touches qui dépassent de la surface de la tablette, en un temps donné. Autrement dit ce dispositif permet d'évaluer la capacité de flexion/extension du poignet et des doigts de la main d'un individu, ainsi que de mesurer la vitesse de ces mouvements dans le temps.

Cependant ce dispositif présente un manque de sensibilité de détection, en particulier quand des personnes atteintes de troubles neuro-musculaires sont concernées. Dans ces cas le dispositif ne peut détecter les appuis trop faibles, réalisés par le patient dont la force musculaire peut être trop faible pour être détectée par ce dispositif.

Un autre inconvénient de ce dispositif connu réside dans le contact entre chaque touche et le capteur associé placé juste au-dessous. Ce contact n'est pas fiable dans la mesure où chaque touche repose par gravité sur un capteur. En cas de vibrations, chocs ou mouvements quelconques du dispositif, le contact est perturbé ce qui fausse les mesures. De plus des frottements importants induisent une usure des pièces en contact ce qui contribue à dérégler les réglages des seuils de détection.

A titre d'arrière plan technologique on connait aussi un article intitulé « A device for testing the intrinsic muscles of the hand » (Journal of Hand therapy octobre 2007, pages 345-350) qui divulgue un dispositif destiné à évaluer la capacité des doigts à s'écarter les uns des autres. Ce dispositif comprend notamment des encoches pour enchâsser chacun des doigts, chaque encoche pouvant coulisser le long d'une rainure prévue à cet effet. L'objectif de ce dispositif est donc différent de celui de la présente invention de même que les moyens mis en oeuvre.

### EXPOSE DE L'INVENTION

L'invention vise à remédier aux inconvénients de l'état de la technique et notamment à proposer un dispositif du type visé ci-dessus qui permette notamment une plus grande précision ainsi qu'une plus grande fiabilité des mesures.

Pour ce faire est proposé un dispositif d'évaluation de la capacité motrice distale des membres supérieurs d'un individu selon la revendication 1.

On évite ainsi les frottements, les jeux et tous les autres mouvements relatifs entre chaque touche et le capteur associé. La fiabilité et la précision des mesures sont grandement améliorés.

Avantageusement chaque capteur de force présente un seuil de détection réglable dont la valeur minimale est de l'ordre de 10 g. Ce choix permet avantageusement de traiter des mesures relatives à des individus très faibles.

De façon intéressante on améliore considérablement la fiabilité des mesures car les touches ne sont quasiment plus sensibles aux vibrations et/ou autres mouvements saccadés et non voulus du dispositif ou du support sur lequel il est posé.

Avantageusement le système de contrôle comprend un moyen de réglage d'au moins un seuil de sensibilité des capteurs. Ainsi l'utilisateur peut facilement adapter les mesures à chaque cas envisagé. De préférence trois seuils sont choisis.

Préférentiellement le moyen de réglage d'au moins un seuil est intégré dans le dispositif.

En outre le dispositif comprend des moyens de transmission des mesures vers au moins un moyen externe de stockage et/ou traitement desdites mesures. Ces moyens de transmission peuvent être filaires ou non.

Selon un mode préféré de réalisation de l'invention, ladite surface principale d'appui fait partie d'un support plat constitué d'au moins un premier élément et apte à intégrer les moyens de mesure et/ou le système de contrôle.

En outre le support plat peut comprendre un deuxième élément juxtaposable au premier élément de façon à augmenter ladite surface d'appui principale. Cette particularité permet d'adapter la taille du dispositif à celle du membre (avant-bras, poignet, main) de l'individu.

De façon intéressante le support plat est constitué en une matière présentant une faible densité telle qu'une mousse de polyéthylène. Un allégement du poids du dispositif facilite son transport et sa maniabilité.

De plus la matière choisie autorise des formes diverses et variées qui combinent la technicité attendue et l'esthétisme d'ensemble du dispositif.

Le dispositif comprend en outre un moyen d'affichage du nombre d'actions comptabilisées et/ou du temps passé ou restant depuis la première séquence d'actions. Préférentiellement le moyen d'affichage est intégré dans le support ; il peut par exemple affleurer avec la surface principale du support afin de constituer un ensemble fonctionnel et esthétique.

Avantageusement, lesdites surfaces d'appui des organes poussoirs sont situées à des altitudes égales ou différentes l'une de l'autre. Le réglage de l'altitude est aisé et très avantageux.

### BREVE DESCRIPTION DES FIGURES

[001] D'autres caractéristiques, détails et avantages de l'invention ressortiront à la lecture de la description qui suit, en référence aux figures annexées, qui illustrent :
- la figure 1, une vue en perspective d'un dispositif selon un premier mode de réalisation;
- la figure 2, une autre vue en perspective d'un dispositif selon un premier mode de réalisation;
- la figure 3, une vue de dessus partielle d'un dispositif; et
- la figure 4 une vue de côté d'un dispositif.

Pour plus de clarté, les éléments identiques ou similaires sont repérés par des signes de référence identiques sur l'ensemble des figures.

### DESCRIPTION DETAILLEE D'UN MODE DE REALISATION

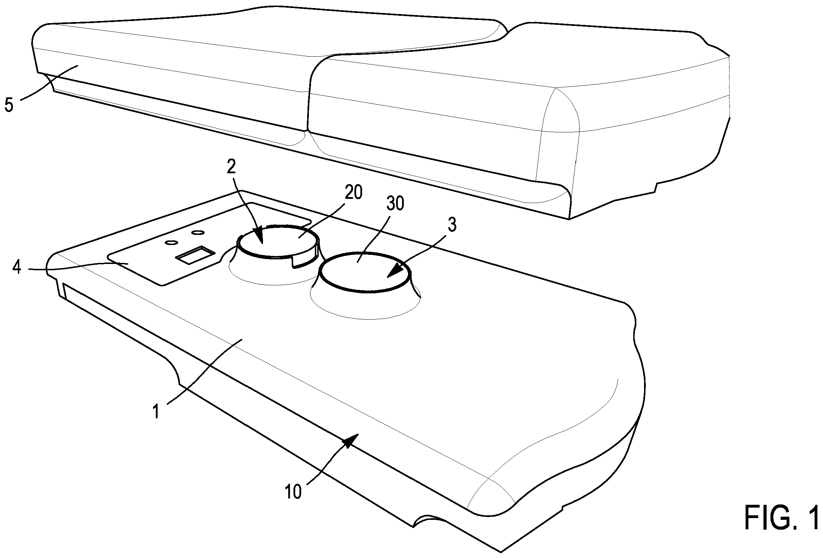

Selon la vue de la figure 1 le dispositif d'évaluation comprend une surface d'appui principale 1 qui est ici de forme globalement rectangulaire et sert d'appui pour tout ou partie d'un membre supérieur d'un individu, à savoir son avant-bras et son poignet. A partir et au-dessus de cette surface d'appui principale 1, un premier 2 et un deuxième 3 organes poussoirs (ou touches) sont prévus et dépassent de ladite surface d'appui 1. Chaque organe présente donc une surface d'appui 20, 30 située dans un plan parallèle et différent de celui de la surface d'appui principale 1. Par ailleurs chaque organe poussoir est couplé avec un capteur de force apte à générer un signal représentatif de la force exercée par l'individu sur la surface d'appui 20, 30 de chaque organe poussoir 2, 3. Par ailleurs un système de contrôle est prévu, couplé notamment aux capteurs de force, afin de recueillir, mettre en forme et traiter les signaux issus des capteurs.

De façon intéressante les capteurs ont été choisis de telle sorte à avoir une sensibilité très faible, de l'ordre de 10g ou bien 30g ou bien 50g par exemple. Il est ainsi possible non seulement de détecter des efforts très faibles mais en outre un réglage peut être réalisé afin d'adapter la sensibilité des mesures au sujet testé.

Concernant les touches 2, 3 en elles-mêmes, elles présentent préférentiellement une surface d'appui 20, 30 de forme circulaire ; avantageusement elles sont fixées par vissage sur chaque capteur d'effort placé juste au-dessous. On privilégie une fixation certes amovible mais rigide des touches 2, 3 sur les capteurs afin que les efforts sur les touches 2,3 puissent être fidèlement reportées sur chaque capteur. Ceci permet donc d'améliorer la fiabilité des mesures.

Par ailleurs une précision de l'ordre de 0.01% caractérise chaque capteur, afin d'avoir un report très précis de chaque effort sur les capteurs. A titre illustratif les capteurs choisis sont des capteurs de pesage de la marque déposée SCAIME, référence AQ10.

Les organes poussoirs 2,3 sont ainsi placés en une position fixe et donnée de la surface principale 1 et ils sont alignés selon une longueur de ladite surface principale 1. L'important est qu'ils soient alignés sensiblement selon la direction longitudinale du membre (avant-bras) placé sur ladite surface principale 1. Si besoin est, des repères visuels peuvent être prévus sur la surface principale afin de montrer le positionnement adéquate de l'avant-bras ; des moyens tels que des sangles peuvent aussi être prévus afin non seulement de positionner mais aussi de maintenir l'avant-bras.

Les organes poussoirs sont de préférence de forme circulaire et ils sont entourés sur leur périphérie par une sorte de cadre circulaire qui s'élève depuis la surface principale 1. Le cadre est avantageusement moulé avec la surface principale 1 et on prévoit une surface de jonction arrondie, non cassante afin que les doigts de l'utilisateur ne puissent se heurter contre une surface présentant une arête vive.

La surface d'appui 20, 30 des organes poussoirs 2,3 peut avantageusement être disposée à une altitude différente de la surface d'appui principale 1. Par ailleurs les organes poussoirs (ou touches) 2, 3 peuvent dépasser de façon différente de la surface d'appui 1. De façon préférée la touche distale 2 (la plus éloignée du patient) est plus haute que la touche proximale 3. Ceci permet de faire exécuter au patient un mouvement de levée de main et de poignet, ce qui sollicite ses extenseurs des doigts et du poignet. A titre illustratif la touche proximale 3 est placée à 1.5 cm de la surface principale 1 tandis que la touche distale 2 peut être placée à 2.5 cm ou encore à 3.5 cm de ladite surface principale 1. Bien entendu la touche distale 2 peut être placée à 1.5 cm de la surface principale 1. D'autres valeurs d'altitudes peuvent être prévues et choisies afin d'augmenter ou au contraire de simplifier la charge de travail musculaire du patient. Le vissage utilisé dans le cadre de l'invention, entre les touches et le capteur associé, permet de régler aisément et précisément l'altitude de l'une au moins des touches 2,3.

Par ailleurs un logiciel associé permet de 'gérer' les séquences d'appui sur les touches : on cherche ainsi à vérifier que l'utilisateur appuie alternativement sur les touches 2, 3. S'il en est autrement, le logiciel arrête le comptage c'est-à-dire le processus d'enregistrement des mesures.

Le système de contrôle, non représenté, comprend un moyen de réglage d'au moins un seuil de sensibilité des capteurs. Cette fonctionnalité permet d'adapter le dispositif à la personne qui doit être évaluée. De préférence ce moyen de réglage est intégré dans le corps du dispositif. Une manette, bouton de commande ou touche sensitive permet à l'utilisateur d'afficher le seuil de sensibilité choisi. De façon préférée trois seuils de sensibilité sont disponibles à savoir 'fort', 'moyen', 'faible'. L'utilisateur choisit l'un parmi ceux-ci, à sa convenance et en fonction du cas envisagé.

Les mesures concernent par exemple le nombre d'appuis réalisés par l'utilisateur successivement sur chacune des touches 2,3, dans un laps de temps donné. Il s'agit donc ici d'une part de compter et de comptabiliser ce nombre d'appuis et d'autre part d'associer un chronomètre à cette opération. Ce test est connu en lui-même ; la présente invention constitue une amélioration notable de ces mesures notamment au niveau de la fiabilité et de la sensibilité.

Comme visible sur les figures, la surface principale d'appui 1 fait partie et/ou est fixée sur un support plat constitué d'au moins un premier élément 10 apte à loger les moyens de mesure et/ou le système de contrôle. Le premier élément 10 est par exemple réalisé en une mousse de polyéthylène dont la densité est faible. Des matériaux à base de polyamide, de polypropylène peuvent être utilisés sans sortir du cadre de l'invention. A titre illustratif une mousse de Plastazote de densité égale à 45 kg/m3 a été choisie avec succès.

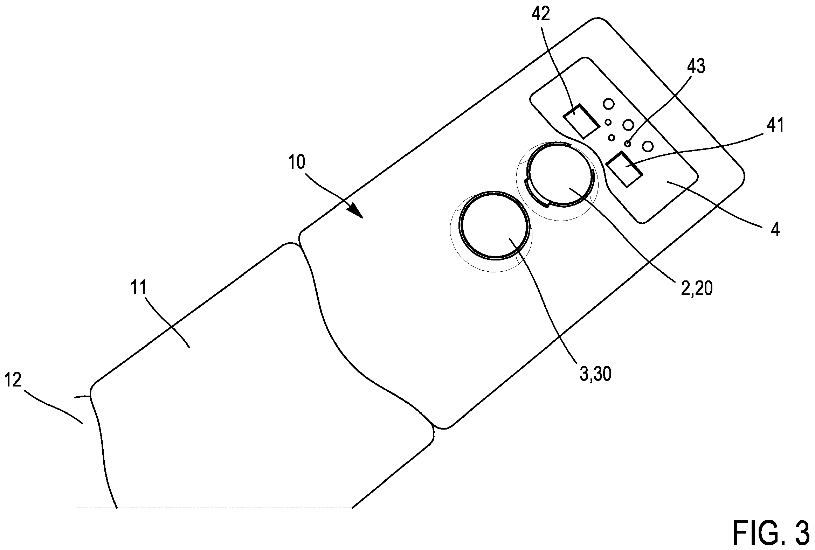

Le premier élément 10 du support peut présenter des formes diverses et modulables. Pour des raisons d'encombrement et/ou de commodité de transport, un deuxième élément 11 peut être prévu, juxtaposable au premier élément 10. La figure 3 illustre une juxtaposition possible, qui permet d'allonger la surface d'appui principale 1 et de l'adapter à la morphologie de l'individu (adulte, enfant). Eventuellement un troisième élément 12 peut être prévu, disposé dans le prolongement des deux premiers. L'homme de métier choisira en fonction des contraintes dimensionnelles imposées par la morphologie du patient évalué.

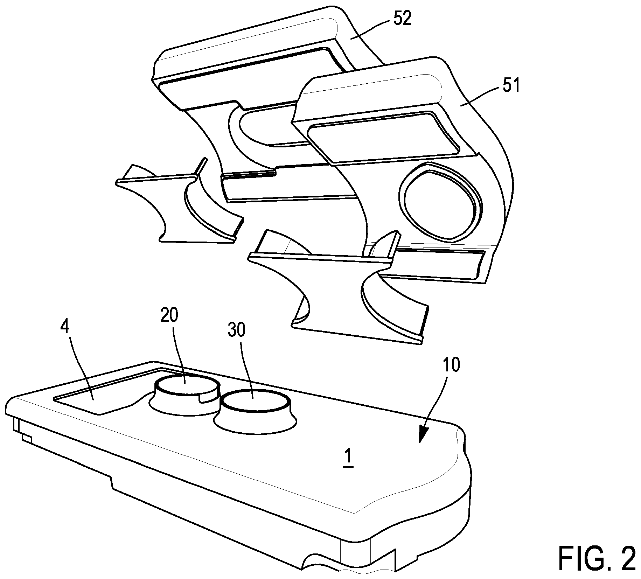

Un couvercle 5; 51,52 peut venir couvrir et protéger la surface principale d'appui 1 munie des touches 2,3. Toute forme, matière peut être envisagée pour le couvercle qui peut être monobloc 5 (figure 1) ou constitué de plusieurs parties 51, 52 (figure 2). Une mallette de transport peut ainsi être conçue, constituée pour partie du support 10 et pour partie d'un couvercle 5 ; 51,52. En outre la forme du couvercle 5 peut être telle qu'elle serve de deuxième élément d'appui 11 lors des mesures. De même pour le troisième élément d'appui 12 qui peut constituer la deuxième partie 52 du couvercle 5. Le couvercle 5 et/ou chaque partie constitutive 51, 52 présente ainsi une double fonction : protection pendant le transport et/ou le stockage du dispositif ; prolongement du premier élément 10 pendant les mesures.

Par ailleurs un moyen d'affichage 4, préférentiellement intégré dans le premier élément 10 du support, est prévu. Il peut comprendre un module d'affichage 41 du nombre d'appuis (ou actions), un module d'affichage 42 du temps écoulé depuis un instant donné ou restant depuis la dernière séquence d'actions ; des témoins lumineux ayant chacun une fonctionnalité donnée (seuil de sensibilité, alimentation ...) peuvent faire partie de l'affichage; un ou plusieurs témoins lumineux 43 correspondant par exemple chacun à un seuil ou niveau de sensibilité des touches 2,3. Ces affichages seront choisies par l'homme de métier afin d'adapter le dispositif aux besoins. Comme visible notamment sur les figures 1,2 et 3 le module d'affichage 4 présente une surface transparente située dans le même plan que la surface d'appui principale 1, ainsi totalement intégrée dans le support plat 10.

Grâce à un logiciel spécifique préférentiellement embarqué, il est possible de régler un nombre fini de seuils de sensibilité ; ceci permet donc d'adapter les mesures aux capacités motrices de l'individu que l'on souhaite évaluer.

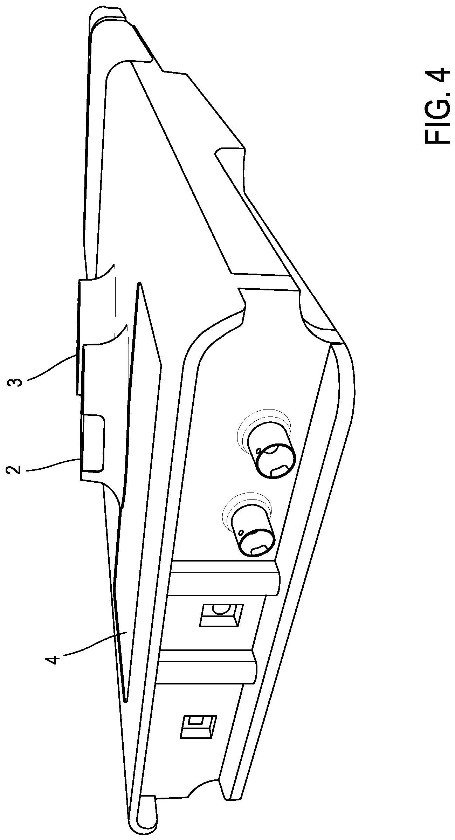

Des moyens de connexion et de transmission des mesures sont prévus, visibles sur la figure 4. Les liaisons peuvent être filaires du type RS232 par exemple, ou bien sans fil. Elles permettent de relier le dispositif à des moyens de calcul, de visualisation, de maintenance... ou autre. Un port USB peut être prévu sur la face avant du dispositif. Il peut être utilisé par exemple pour mettre à jour le logiciel embarqué.

## Revendications

1. Dispositif d'évaluation de la capacité motrice distale des membres supérieurs d'un individu, le dispositif comprenant une surface d'appui principale (1) pour tout ou partie d'un membre supérieur, un moyen de mesure comprenant deux organes poussoirs (2,3) destinés à être alignés sensiblement selon la direction longitudinale du membre en place sur ladite surface d'appui principale, chacun des organes poussoirs doté d'une surface d'appui (20, 30) située dans un plan parallèle et différent de ladite surface d'appui principale (1) et chacun couplè à un capteur de force apte à générer un signal relatif à la force exercée par l'individu sur ladite surface d'appui, et un système de contrôle couplé au moyen de mesure, ledit système de contrôle permettant de compter un nombre d'appuis alternatifs sur chaque surface d'appui (20, 30) **caractérisé en ce que** lesdits organes poussoirs (2, 3) sont liés au capteur de force par vissage occupent une position fixe et immobile sur la surface d'appui principale (1), sont destinés à être alignés sensiblement selon la direction longitudinale du membre en place sur ladite surface et **en ce que** chaque capteur de force présente une précision de l'ordre de 0.01%.

2. Dispositif d'évaluation de la capacité motrice distale des membres supérieurs d'un individu selon la revendication 1 **caractérisé en ce que** chaque capteur de force présente un seuil de détection réglable dont la valeur minimale est de l'ordre de 10g.

3. Dispositif d'évaluation de la capacité motrice distale des membres supérieurs d'un individu selon l'une quelconque des revendications précédentes **caractérisé en ce que** le système de contrôle comprend un moyen de réglage d'au moins un seuil de sensibilité des capteurs.

4. Dispositif d'évaluation de la capacité motrice distale des membres supérieurs d'un individu selon la revendication 3 **caractérisé en ce que** ledit moyen de réglage est intégré dans le dispositif.

5. Dispositif d'évaluation de la capacité motrice distale des membres supérieurs d'un individu selon l'une quelconque des revendications précédentes **caractérisé en ce qu'**il comprend des moyens de transmission des mesures vers au moins un moyen externe de stockage et/ou de traitement desdites mesures.

6. Dispositif d'évaluation de la capacité motrice distale des membres supérieurs d'un individu selon l'une des revendications précédentes **caractérisé en ce que** ladite surface principale d'appui (1) fait partie d'un support plat constitué d'au moins un premier élément (10) et apte à intégrer les moyens de mesure et/ou le système de contrôle.

7. Dispositif d'évaluation de la capacité motrice distale des membres supérieurs d'un individu selon la revendication 6 **caractérisé en ce que** le support plat comprend un deuxième élément (11) juxtaposable au premier élément (10) de façon à augmenter ladite surface d'appui principale.

8. Dispositif d'évaluation de la capacité motrice distale des membres supérieurs d'un individu selon la revendication 6 **caractérisé en ce que** le support plat (10) est constitué en un matériau à base de polyamide, de polypropylène ou d'une mousse de polyéthylène.

9. Dispositif d'évaluation de la capacité motrice distale des membres supérieurs d'un individu selon l'une quelconque des revendications précédentes **caractérisé en ce qu'**il comprend en outre un moyen d'affichage (4) du nombre d'actions comptabilisées et/ou du temps passé ou restant depuis la première séquence d'actions.

10. Dispositif d'évaluation de la capacité motrice distale des membres supérieurs d'un individu selon la revendication précédente **caractérisé en ce que** ledit moyen d'affichage (4) est intégré dans ledit support (10).

11. Dispositif d'évaluation de la capacité motrice distale des membres supérieurs d'un individu selon l'une quelconque des revendications précédentes **caractérisé en ce que** lesdites surfaces d'appui (20, 30) des organes poussoirs (2, 3) sont situées à des altitudes égales ou différentes l'une de l'autre.

## Patentansprüche

1. Vorrichtung zur Bewertung der distalen motorischen Fertigkeiten der oberen Extremitäten einer Person, wobei die Vorrichtung eine Hauptstützfläche (1) für ein gesamtes oder einen Teil einer oberen Extremität umfasst, wobei das Messmittel zwei Schuborgane (2, 3) umfasst, die dazu bestimmt sind, deutlich gemäß der Längsrichtung der auf der Hauptstützfläche vorhandenen Extremität gefluchtet zu sein, wobei jedes der Schuborgane mit einer Stützfläche (20, 30) versehen ist, die in einer zu der genannten Hauptstützfläche (1) parallelen und unterschiedlichen Ebene angeordnet ist und jeweils mit einem Kraftsensor gekoppelt ist, der geeignet ist, ein Signal relativ zu der von der Person auf die genannte Stützfläche ausgeübten Kraft zu generieren, und ein Kontrollsystem, das mit dem Messmittel gekoppelt ist, wobei das genannte Kontrollsystem das Zählen einer Anzahl von alternativen Aufstützungen auf jeder Stützfläche (20, 30) erlaubt, **dadurch gekennzeichnet, dass** die genannten Schuborgane (2, 3) mit dem Kraftsensor per Verschraubung verbunden sind, eine feste und unbewegliche Position auf der Hauptstützfläche (1) einnehmen, dazu bestimmt sind, deutlich gemäß der Längsrichtung der Extremität gefluchtet zu sein, die auf der genannten Fläche vorhanden ist, und dass jeder Kraftsensor eine Präzision in der Größenordnung von 0,01 % aufweist.

2. Vorrichtung zur Bewertung der distalen motorischen Fertigkeiten der oberen Extremitäten einer Person gemäß Anspruch 1, **dadurch gekennzeichnet, dass** jeder Kraftsensor einen einstellbaren Detektionsschwellenwert aufweist, dessen Mindestwert in der Größenordnung von 10 g liegt.

3. Vorrichtung zur Bewertung der distalen motorischen Fertigkeiten der oberen Extremitäten einer Person gemäß irgendeinem der voranstehenden Ansprüche, **dadurch gekennzeichnet, dass** das Kontrollsystem ein Einstellmittel wenigstens eines Sensibilitätsschwellenwertes der Sensoren umfasst.

4. Vorrichtung zur Bewertung der distalen motorischen Fertigkeiten der oberen Extremitäten einer Person gemäß Anspruch 3, **dadurch gekennzeichnet, dass** das genannte Einstellmittel in die Vorrichtung eingebaut ist.

5. Vorrichtung zur Bewertung der distalen motorischen Fertigkeiten der oberen Extremitäten einer Person gemäß irgendeinem der voranstehenden Ansprüche, **dadurch gekennzeichnet, dass** es Übertragungsmittel der Messungen zu wenigstens einem externen Lager- und / oder Verarbeitungsmittel der genannten Messungen umfasst.

6. Vorrichtung zur Bewertung der distalen motorischen Fertigkeiten der oberen Extremitäten einer Person gemäß einem der voranstehenden Ansprüche, **dadurch gekennzeichnet, dass** die genannte Hauptstützfläche (1) Teil eines flachen Trägers ist, der aus wenigstens einem ersten Element (10) gebildet und geeignet ist, die Messmittel und / oder das Kontrollsystem aufzunehmen.

7. Vorrichtung zur Bewertung der distalen motorischen Fertigkeiten der oberen Extremitäten einer Person gemäß Anspruch 6, **dadurch gekennzeichnet, dass** der flache Träger ein zweites Element (11) umfasst, das neben dem ersten Element (10) derart angeordnet ist, dass die genannte Hauptstützfläche erhöht wird.

8. Vorrichtung zur Bewertung der distalen motorischen Fertigkeiten der oberen Extremitäten einer Person gemäß Anspruch 6, **dadurch gekennzeichnet, dass** der flache Träger (10) aus einem Material auf Polyamid-, Polypropylen- oder einer Polyethylenschaum-Basis gebildet ist.

9. Vorrichtung zur Bewertung der distalen motorischen Fertigkeiten der oberen Extremitäten einer Person gemäß irgendeinem der voranstehenden Ansprüche, **dadurch gekennzeichnet, dass** es darüber hinaus Anzeigemittel (4) der Anzahl der verbuchten Aktionen und / oder der seit der ersten Sequenz von Aktionen verstrichenen oder verbliebenen Zeit umfasst.

10. Vorrichtung zur Bewertung der distalen motorischen Fertigkeiten der oberen Extremitäten einer Person gemäß dem voranstehenden Anspruch, **dadurch gekennzeichnet, dass** das genannte Anzeigemittel (4) in den genannten Träger (10) eingebaut ist.

11. Vorrichtung zur Bewertung der distalen motorischen Fertigkeiten der oberen Extremitäten einer Person gemäß irgendeinem der voranstehenden Ansprüche, **dadurch gekennzeichnet, dass** die genannten Stützflächen (20, 30) der Schuborgane (2, 3) in Höhen angeordnet sind, die jeweils gleich oder voneinander unterschiedlich sind.

## Claims

1. A device for evaluating the distal motor skills of the upper limbs of a person, the device comprising a main support surface (1) for all or part of an upper limb, a measuring means comprising two pusher members (2, 3) intended to be aligned substantially in the longitudinal direction of the limb in place on said main bearing surface, each of the pusher members provided with a bearing surface (20, 30) situated in a plane parallel to and different from said main bearing surface (1) and each coupled to a force sensor able to generate a signal relating to the force exerted by the person on the bearing surface, and a control system coupled to the measuring means, said control system making it possible to count a number of alternative pressings on each bearing surface (20, 30), **characterised in that** said pusher members (2, 3) are connected to the force sensor by screwing, occupying a fixed and immobile position on the main bearing surface (1), are intended to be aligned substantially in the longitudinal direction of the limb in place on said surface and **in that** each force sensor has a precision of around 0.01%.

2. A device for evaluating the distal motor skills of the upper limbs of an individual according to claim 1, **characterised in that** each force sensor has an adjustable detection threshold, the minimum value of which is around 10 g.

3. A device for evaluating the distal motor skills of the upper limbs of an individual according to any of the preceding claims, **characterised in that** the control system comprises a means for adjusting at least one sensitivity threshold of the sensors.

4. A device for evaluating the distal motor skills of the upper limbs of an individual according to claim 3, **characterised in that** said adjustment means is integrated in the device.

5. A device for evaluating the distal motor skills of the upper limbs of an individual according to any of the preceding claims, **characterised in that** it comprises means for transmitting the measurements to at least one external means for storing and/or processing said measurements.

6. A device for evaluating the distal motor skills of the upper limbs of an individual according to any of the preceding claims, **characterised in that** said main bearing surface (1) forms part of a flat support consisting of at least one first element (10) and able to integrate the measuring means and/or the control system.

7. A device for evaluating the distal motor skills of the upper limbs of an individual according to claim 6, **characterised in that** the flat support comprises a second element (11) juxtaposable with the first element (10) so as to increase said main support surface.

8. A device for evaluating the distal motor skills of the upper limbs of an individual according to claim 6, **characterised in that** the flat support (10) is formed from a material based on polyamide, polypropylene or a polyethylene foam.

9. A device for evaluating the distal motor skills of the upper limbs of an individual according to any of the preceding claims, **characterised in that** it further comprises a means (4) for displaying the number of actions counted and/or the time passed or remaining since the first sequence of actions.

10. A device for evaluating the distal motor skills of the upper limbs of an individual according to the preceding claim, **characterised in that** said display means (4) is integrated in said support (10).

11. A device for evaluating the distal motor skills of the upper limbs of an individual according to any of the preceding claims, **characterised in that** said bearing surfaces (20, 30) of the pusher members (2, 3) are situated at heights equal to or different from one another.
